Europäisches Patentamt

European Patent Office

Office européen des brevets

(19)

(11) Publication number: **0 159 852**
A2

# EUROPEAN PATENT APPLICATION

(12)

(21) Application number: **85302470.1**

(22) Date of filing: **09.04.85**

(51) Int. Cl.⁴: **A 61 K 9/20**

(30) Priority: **16.04.84 US 600809**

(43) Date of publication of application: **30.10.85**
**Bulletin 85/44**

(84) Designated Contracting States: **AT BE CH DE FR GB IT LI LU NL SE**

(71) Applicant: **MALLINCKRODT, INC., 675 McDonnell Boulevard P.O. Box 5840, St. Louis Missouri 63134 (US)**

(72) Inventor: **Salpekar, Anil, 1421 Joywood Drive, Creve Coeur, MO 63146 (US)**
Inventor: **Haag, Thomas E., 624 Fox Creek Ct., Crestwood MO 63121 (US)**

(74) Representative: **Eyles, Christopher Thomas et al, BATCHELLOR, KIRK & EYLES 2 Pear Tree Court Farringdon Road, London, EC1R 0DS (GB)**

(54) **Directly compressible pharmaceutical compositions.**

(57) A compressible pharmaceutical composition is disclosed comprising an interactive ingredient such as codeine, a compound such as polyvinylpyrrolidone which acts as a binder and a filler such as lactose. The composition is granulated and can thereafter be dry blended with other active ingredients such as acetaminophen, aspirin, ibuprofen, sodium naproxen or the like. The mixture may then be tableted in the usual manner to produce a tablet having physical properties comparable to tablets in which all the active ingredients are wet granulated together, yet avoiding the intimate contact between the ingredients which can result in undesirable interaction and adversely affect the stability of the active ingredients.

EP 0 159 852 A2

ACTORUM AG

# DIRECTLY COMPRESSIBLE PHARMACEUTICAL COMPOSITIONS

This invention lies in the art of pharmaceuticals and more particularly can be found in the field of tablet compositions for oral administration. Specifically, the invention comprises a composition of a reactive therapeutic agent which, when admixed with other analgesics and excipients, minimizes contact between the active ingredients thus producing chemically and physically more stable dosage forms.

Heretofore, analgesic compositions have been prepared using various wet granulation processes which provide for admixing the active ingredients with various excipients. For certain pharmaceutical mixtures of two or more active ingredients, however, wet granulation is not an ideal process for making tablets. Besides being both costly and time-consuming, wet granulation of a mixture allows the active ingredients to be in intimate physical contact, as there is no mechanism for effectively isolating individual ingredient particles. If the ingredients are interactive, this physical contact may, in a surprisingly short period of time after tableting, result in loss of chemical potency due to degradative chemical reaction. Many analgesics are interactive. Thus, for example, a mixture of codeine or codeine salt and another analgesic such as aspirin or acetaminophen will interact if tableted using a wet granulation technique.

The water used in wet granulation processes also contributes to product instability. Residual moisture is almost impossible to remove from granulated material without the application of high drying temperatures which could in itself degrade the active ingredients. The residual moisture can, over a period of time, react with and thus reduce the potency of the active ingredients. Further, one or more of the active ingredients may be water soluble to an extent such that moisture in the compressed tablet causes partial solubilization which in turn increases the physical contact between the active ingredients.

Thus, there is a need in the art for a pharmaceutical formulation process which will eliminate the need for wet granulation when two or more pharmaceutically interactive compounds are combined. Examples of such combinations are mixtures of codeine or codeine salts with other analgesic agents such as acetaminophen, aspirin, ibuprofen, sodium naproxen or the like.

We have discovered that, by separately granulating at least one of the active compounds, such as a codeine salt, with a binder and a filler, a second analgesic compound in a suitable form can be added to the preparation and the mixture directly tableted. Intimate contact between the two active ingredients is reduced, as the codeine or other granulated compound is isolated in the granular matrix.

Generally stated, the present invention provides a method of producing a stable, directly tabletable composition of two or more pharmaceutically active ingredients comprising: blending one of the active ingredients with a binder and a filler; wet granulating the mixture so produced in the presence of a solvent; drying the granulated mixture; sizing the granulated mixture; and blending the mixture with one or more other pharmaceutically active ingredients.

Certain pharmaceutical compounds well known to interact with other active ingredients when combined in a single tablet composition include, for example, codeine and its salts, which, in conjunction with other non-steroidal analgesics such as acetaminophen, aspirin, ibuprofen, sodium naproxen and the like, react in such a way as to cause discoloration of the product and adversely affect the stability of the active ingredients. This interactive effect is greatly enhanced when the active ingredients are brought together in intimate contact with each other by wet granulating with a binder solution.

By providing a process whereby an interactive pharmaceutical is independently granulated with an aqueous or non-aqueous solvent and thereafter dryblended with the remaining active ingredient or ingredients (which may or may not themselves be granulated), the present invention has been found to provide sufficient isolation of the interactive compounds so that enhanced chemical and physical stability is achieved. The use of a non-aqueous solvent, such as an alcohol, is preferred inasmuch as it eliminates the problems inherent in the use of water as a solvent, namely, solubilization of the active ingredients and their reaction with the water.

In the process of the invention, the codeine or other interactive pharmaceutical is wet granulated with a binder such as polyvinylpyrrolidone and a filler such as lactose. While the discussion hereinafter refers primarily to codeine and its salts, it is to be understood that the invention is equally applicable to the tableting of compositions containing other interactive pharmaceuticals, such as oxycodone, hydrocodone, and other narcotic and non-narcotic analgesic agents.

Codeine (7,8-didehydro-4,5 -epoxy-3-methoxy-17-methylmorphinan-6 -ol), as used in the present invention, is preferably provided in finely divided form, i.e., the codeine is preferably of small particle size. For use in the present invention, preferably all of the codeine particles will pass through a 100 mesh screen, more preferably most will pass through a 200 mesh screen, and most preferably all will pass through a 200 mesh screen. Preferably, the codeine is in the form of a physiologically acceptable salt, such as codeine phosphate, codeine sulfate or the like, which are known to provide chemical stability.

The terms "direct tableting" or "compressible" and terms of like import, as used herein, mean that the composition can be formed into a tablet using well known tableting apparatus and processes with only the addition of inert excipients to aid in tablet manufacture (e.g., tablet lubricants). As used herein, the term "Kp" means kiloponds, a well known unit of force for expressing hardness or crushing strength of pharmaceutical tablets when such hardness is determined on a Schleuniger Tablet Hardness Tester.

Binders can be chosen from the pharmaceutically acceptable synthetic and natural polymers (e.g., acacia, gelatin, sucrose, starch, alginic acid, methylcellulose, sodium carboxymethylcellulose, ethyl cellulose, polyvinylpyrrolidone). Polyvinylpyrrolidone is preferred. Suitable polyvinylpyrrolidone is characterized by the raw material specifications outlined in the USP XX monograph for Povidone.

5

Fillers can be chosen from pharmaceutically acceptable inert ingredients such as starches, celluloses, sugars, or inorganic excipients such as calcium phosphate. The filler preferred in this composition is lactose, USP. Lactose is characterized by the raw material specifications outlined in NF XV.

Generally the codeine salt or other active ingredient to be granulated is present in amounts up to about 80 parts per 100 parts by weight of the granulating composition, preferably from 5 to about 80 parts. The binder can range from about 2 to about 10 parts while the filler is from about 18 to about 85 parts per 100 parts of the granulating composition. The non-aqueous solvent is added to the granulation process in amounts sufficient to effect granulation. Any pharmaceutically acceptable non-aqueous solvent may be used. While aqueous solvents can be used, non-aqueous solvents are preferred for the reasons given above. Of the latter, absolute ethyl alcohol is most preferred. The preferred amount of active ingredient is about 50 parts per 100 parts by weight granulating composition. About 4 parts of binder and about 46 parts of filler are preferred.

A preferred embodiment thus includes the following components in the amounts indicated:

| COMPONENTS | APPROXIMATE AMOUNTS |
|---|---|
| Codeine salt | 5-80 |
| Lactose | 18-85 |
| Polyvinylpyrrolidone | 2-10 |
| Alcohol 3A (absolute ethyl alcohol) | quantity sufficient for granulation |

The amounts shown are in parts per 100 parts by weight of the composition.

A preferred embodiment composition is as follows, wherein the amounts given are in parts per 100 parts by weight of the composition.

| COMPONENTS | APPROXIMATE AMOUNTS |
| --- | --- |
| Codeine Phosphate, USP | 50 |
| Lactose, USP (Fast Flow) | 46 |
| Polyvinylpyrrolidone, (USP) | 4 |
| Alcohol 3A (absolute ethyl alcohol) | quantity sufficient for granulation |

Such compositions, which are themselves directly compressible, can be combined with other active ingredients and inert excipients by, for example, dry blending, and they can thereafter be directly compressed into tablets. The other active ingredients can be, for example, any of the previously mentioned analgesic agents, i.e., non-steroidal analgesics including acetaminophen, aspirin, ibuprofen, sodium naproxen and the like. Preferably, the other active ingredient(s) to be mixed with the granulated codeine salt composition are themselves separately granulated with a binder and filler, in a manner similar to that described above, to produce particles of similar size to the granulated codeine-containing particles. This ensures that the final composition will be directly compressible and provides additional isolation of the interactive ingredients.

The compositions of this invention are preferably made by the method mentioned above, which includes wet granulating the codeine phosphate, lactose, and one-half the amount of polyvinylpyrrolidone with an absolute ethyl alcohol solution containing the remaining polyvinylpyrrolidone, followed by classification, drying and blending with the other active ingredients.

The preferred apparatus for granulation is a twin-shell blender with liquid feed bar or planetary mixer of suitable capacity. The following general procedure illustrates the granulation process:

Planetary mixer - the granulating solution (absolute ethyl alcohol plus one-half the amount of polyvinylpyrrolidone) is gradually added to the blend containing codeine phosphate, lactose, and polyvinylpyrrolidone until a satisfactory granulation is achieved. The blend is constantly being mixed during the addition of the granulating solution.

Patterson-Kelley Blender (twin-shell) - the granulating solution (absolute ethyl alcohol plus one-half the amount of polyvinylpyrrolidone) is gradually added to the blend via the liquid-feed tube until a satisfactory granulation is achieved.

After granulation, the composition is wet-screened, dried in a suitable dryer such as a tray drying oven, fluid-bed dryer or vacuum oven and dry screened to the proper particle size using a Stokes granulator or other suitable wet milling equipment. If a drying oven is used, it is operated at a temperature of from about 50°C to about 54°C. The material is dried until a moisture level below 4% is obtained.

When a fluid-bed dryer is used, an inlet temperature of about 45°C is used and the material is dried until a moisture level below 4% is obtained.

When a vacuum oven is used, a temperature of about 45°C-50°C is used and the material is dried until a moisture level below 4% is obtained.

The following examples illustrate the methods and compositions of the invention. Examples 1-7 produced

0159852

8

directly compressible (DC) compositions which, when dry blended with other active ingredients and excipients and tableted, resulted in tablets having excellent shelf life with little or no discoloration or chemical degradation. Examples 8 and 9 are comparative, in which the methods and compositions of the invention are evaluated against those of the prior art.

### EXAMPLE 1

Approximately 6,000 gm codeine phosphate, 5520 gm fast-flow lactose, and 240 gm polyvinylpyrrolidone were blended in a twin-shell blender for one-half hour, followed by transfer to a planetary mixer mixing bowl. Approximately 240 gm polyvinylpyrrolidone, mixed in 2.5 liters absolute ethyl alcohol using a Brookfield Counter-Rotating Mixer were added to the mixing bowl. The mixer was then run on slow speed and a granulation procedure begun. An additional 460 cc of alcohol was added to the mixture. After granulation, the mixture was wet-milled with a Fitz mill and dried at 54°C in a tray-drying oven. The granulation was then dry-milled using a Fitz mill.

### EXAMPLE 2

Approximately 300 gm codeine phosphate, 276 gm fast-flow lactose, and 12 gm polyvinylpyrrolidone were mixed in a twin-shell blender for 30 minutes, after which the mixture was transferred to a planetary mixing bowl. Approximately 12 gm polyvinylpyrrolidone in 150 cc absolute ethyl alcohol was added to the mixing bowl, followed by an additional 90 cc of alcohol. The mixture was then wet-milled with a Stokes granulator, followed by drying at 52°C in a tray-drying oven. The mixture was then dry-milled, using a Stokes granulator.

9

### EXAMPLE 3

Same as Example 2 except hydroxypropyl methylcellulose was substituted for polyvinylpyrrolidone and the granulating solution was 95% alcohol/5% water.

### EXAMPLE 4

Same as Example 2 except 5% ethylcellulose was substituted for the 4% polyvinylpyrrolidone. Thus, only 45% fast-flow lactose was used in the granulation.

### EXAMPLE 5

Same as Example 2 except partially gelatinized starch was substituted for fast-flow lactose and the material was dried at 50°C in a vacuum oven.

### EXAMPLE 6

Same as Example 2 except codeine sulfate was substituted for codeine phosphate.

### EXAMPLE 7

Approximately 600 gm codeine phosphate, 552 gm fast-flow lactose, and 48 gm polyvinylpyrrolidone were dry blended in a twin-shell blender having a liquid feed bar for 25 minutes. Approximately 400 cc of absolute ethyl alcohol was added slowly through the liquid feed tube. This was followed by wet-milling with a Stokes granulator, and drying at 50°C in a tray-drying oven. A Stokes granulator was used for dry-milling to produce the final product.

### EXAMPLE 8

Codeine salts and ibuprofen were mixed together with a binder and filler and the mixture was wet granulated, with water in one instance, and with

10

alcohol in another instance. In both instances, discoloration occurred rapidly during the granulating and drying process.

Tablets were then prepared from a mixture of ibuprofen granules and directly compressible codeine phosphate granules which had been separately produced. The tablets showed excellent color stability even after 3 months storage at 50°C. Even though DC-codeine sulfate was shown to be somewhat less stable than DC-codeine phosphate, it is still more color stable than the wet granulation of ibuprofen and codeine salts together. For these tableting studies, the ibuprofen granulation was derived from commercially obtained Motrin® (Upjohn) tablets.

EXAMPLE 9

Direct compression codeine phosphate was prepared as in Example 1 using first water and then absolute ethyl alcohol as the granulating solution. Visible discoloration appeared in the water granulated product after overnight drying at 50°C. The alcohol granulated product remained white.

Aspirin/DC-codeine phosphate tablets with the following composition were prepared:

A) Tablet Composition

| | |
|---|---|
| Dow #40 XLS Aspirin | 325.00 mg |
| DC Codeine Phosphate (Example 1, alcohol granulated) | 120.00 mg |
| Cab-O-Sil | 1.65 mg |
| Starch 1500 (LM) | 59.50 mg |
| Avicel PH 101 | 59.50 mg |
| Stearic Acid | 16.00 mg |
| Total Weight | 581.65 mg |

11

B)    Blending

All components, except stearic acid were added
to a V-blender and blended for 20 minutes.
Stearic acid was then added and the mixture was
blended an additional 10 minutes.

C)    Tablet Press

Manesty B3B, 16 Station

D)    Tooling

7/16" Standard Concave

E)    Tablet Characteristics

| | |
|---|---|
| Hardness | 9.9 kp |
| Disintegration Time: | 2:50-3:30 |
| Codeine Phosphate Content | 10 tablets, |
| Uniformity Range: | 95-101% |
| Maximum Hardness: | >20 kp |

The aspirin/DC-codeine phosphate (water
granulated) tablets turned yellow after 2 weeks storage
at 50°C.  The aspirin/DC-codeine phosphate (alcohol
granulated) tablets remained white after 3 months
storage at 50°C.  After 3 months storage in
polyethylene bottles with black phenolic closures at
37°C/80% relative humidity, the aspirin/DC-codeine
phosphate (alcohol granulated) tablets show improved
color stability over commercially available 325 mg
aspirin/codeine phosphate tablets.

The best mode contemplated for carrying out this
invention has been set forth in the above description, for example, the way of setting forth preferred materials and operating conditions, including but not limited to preferred ranges and values of amounts and other non-obvious variables material to successfully practice the invention in the best way contemplated at the time of executing this patent application.

It is understood that the foregoing detailed description is given merely by way of illustration and that many modifications may be made therein without departing from the spirit or scope of the present invention.

CLAIMS

1. A method of producing a stable, directly tabletable composition of two or more pharmaceutically active ingredients which are interactive with each other comprising:

blending one of the active ingredients with a binder and a filler;

wet granulating the mixture so produced in the presence of a solvent;

drying the granulated mixture;

sizing the granulated mixture; and

blending the mixture with one or more other pharmaceutically active ingredients.

2. The method of claim 1, wherein the one or more other pharmaceutically active ingredients are also separately granulated with a binder and a filler.

3. The method of claim 1 or 2, wherein the solvent is non-aqueous.

4. The method of claim 3, wherein the solvent is anhydrous ethyl alcohol.

5. The method of any one of the preceding claims wherein the first active ingredient is a codeine salt.

6. The method of claim 5 wherein the first active ingredient is codeine phosphate.

7. The method of any one of the preceding claims wherein the one or more other active ingredients are selected from the group consisting of non-steroidal analgesic agents.

8.     The method of claim 7, wherein the one or more other active ingredients are selected from the group consisting of acetaminophen, aspirin, ibuprofen and sodium naproxen.

9.     A stable, directly tabletable composition of two or more pharmaceutically active ingredients produced by the method of any one of the preceding claims.

10.     The composition of claim 9, wherein the codeine salt is codeine phosphate and the one or more other active ingredients are selected from the group consisting of acetaminophen, aspirin, ibuprofen, and sodium naproxen.